# EUROPEAN PATENT APPLICATION

(11) **EP 3 811 958 A1**
(43) Date of publication of application: **28.04.2021**
(21) Application number: 19823391.8
(22) Date of filing: 21.06.2019
(51) Int. Cl.: A61K 36/48, A61P 9/14

(54) **COMPOSITION FOR THE PREVENTION AND/OR TREATMENT OF HAEMORRHOIDS**

(30) Priority: 21.06.2018 ES 201830606
(71) Applicant: Laboratorios Abbap Pharma S.L., 11540 Sanlúcar de Barrameda, Cádiz (ES)
(72) Inventor: BARES LÓPEZ, Lydia, 11011 Cádiz (ES); BARES DOMÍNGUEZ, Miguel Ángel, 11011 Cádiz (ES)
(74) Representative: Elzaburu S.L.P.
(86) International application number: PCT/ES2019/070431
(87) International publication number: WO 2019/243651

(57) **Abstract**

The present invention relates to a composition for the prevention and/or treatment of haemorrhoids comprising an extract from *Ceratonia siliqua* wood, and a method for preparing same.

## Description

### BACKGROUND

Haemorrhoids, also known as piles, are swollen veins in the anus and lower part of the rectum, similar to varicose veins. Haemorrhoids are very common; almost three quarters of adults suffer from haemorrhoids occasionally. Sometimes they do not show symptoms. However, they can sometimes cause itchiness, discomfort, and bleeding. In general haemorrhoids are treated by topical remedies with witch hazel or a numbing agent, suppositories with hydrocortisone or oral anti-inflammatories. Patches with natural ingredients for the treatment of haemorrhoids have been described in documents CN106913674, CN105920174, CN103919916 and WO2004069323 A1. ES2549703 describes a natural composition for treating haemorrhoids comprising virgin olive oil and carob pods for the treatment of both external and internal haemorrhoids, as well as a method for preparing the composition that comprises placing the olive oil and chopped carob pods in a vessel, allowing the mixture to fry at low heat, cooling, and sifting. Said composition is applied directly on the affected area. ES2549703 does not disclose anything regarding the use of the roots, wood and/or leaves of the carob tree, only of the fruit.

The health benefits of the carob tree (*Ceratonia siliqua*) have been described in numerous publications. in 2014 Durazzo et al. (Food Chem. 2014, 153, 109) found antioxidant properties in flour from carob seeds, of particular interest to the food industry. Benchikh et al. (Ind. Crops. Prod. 2014, 60, 98) found an excellent source of natural antioxidants in extracts of unripe *Ceratonia siliqua* that can be used in the cosmetics, pharmaceutical and food industries. For example, Rached *et al.* used the extract from carob pulp for yoghurt in encapsulated and free forms, the latter form showing a greater antioxidant power (Food Funct., 2016, 7, 1319). Amessis-Ouchemoukh *et al.* showed that extracts of *Ceratonia siliqua* are highly effective in the neutralisation of free radicals (Ind. Crops. Prod. 2017, 95, 6). The extracts of the plant with anti-ageing properties are used in the cosmetics industry (CN105640854; KR20150060004; KR101339915; KR20130113729; KR10080155).

In addition, Meziani *et al.* found antimicrobial compounds in leaves and sheaths of *Ceratonia siliqua* for the bacterium *Pectobacterium atrosepticum,* which causes potato rot (Microb. Pathogenesis 2015, 78, 95). Simultaneously, Hsouna *et al.* identified antimicrobial activity in essential oil of *Ceratonia siliqua* sheaths against *Listeria monocytogenes* in beef mincemeat (Int. J. of Food Microbiol., 2011, 148, 66).

Likewise, it has been described that carob pulp contains polyphenols useful for the treatment of diabetes in humans (S. Gruendel, A.L. Garcia, B. Otto, K. Wagner, M. Bidlingmaier, L. Burget, et al., Br. J. Nutr. 2007, 98, 1170), as an antitumoural treatment (Custodio et al., Rec. Nat. Prod., 2013, 7, 225), with hepatoprotective and nephroprotective effect (Hsouna et al., Food Chem. Toxicol., 2011, 49, 3183), or for heart diseases (Nasar-Abbas et al., Compr. Rev. Food Sci. Food Saf., 2016, 15, 63).

Carvalho *et al.* performed a study that found that raw carob sheaths were used as raw material in the production of succinic acid with a biological base (Bioresource Technol., 2016, 218, 491).

Rtibi et al. found properties against chronic inflammation and oxidative stress in *Ceratonia siliqua* (RSC Adv., 2016, 6, 65483). At the same time, US2016310552 describes the use of carob extract together with green tea and white bean extracts for the treatment and prevention of irritable bower syndrome.

Asgari et al. (The Natural Products Journal, 2012, 2, 1) described that carob has antidiarrhoeic properties, acting not only in the detoxification and constipation, but also providing a calorie-rich food source. WO2011089012 describes a method for producing a medicinal product for diarrhoea from the extract from the fruit of *Ceratonia siliqua.*

There is therefore a need for a remedy for the effective prevention and/or treatment of haemorrhoids with a convenient and painless application.

### DESCRIPTION OF THE INVENTION

The present invention provides an effective remedy for haemorrhoids based on natural ingredients, which can also be applied in a painless and extremely convenient manner, directly on the skin and far from the affected area, preventing the need for suffering due to contact during the application of the remedy.

The inventors of the present invention have found that transdermal application of a pharmaceutical composition comprising an extract from the wood, leaves, and/or roots of the carob tree (*Ceratonia siliqua*), is highly efficient for preventing and or treating haemorrhoids.

In a first aspect the present invention relates to a composition comprising an extract from the wood, leaves, and/or roots of *Ceratonia siliqua* for the prevention and/or treatment of haemorrhoids. In a preferred embodiment, the extract is from wood of *Ceratonia siliqua.* In another preferred embodiment, the extract is from leaves of *Ceratonia siliqua.* In a preferred embodiment, the extract is from wood and leaves of *Ceratonia siliqua.* Other preferred embodiments can comprise an extract from the roots of *Ceratonia siliqua.* The extract of the present invention can be obtained in many ways known to a person skilled in the art, such as by pressing, maceration, absorption or distillation. An example of an extract from leaves of *Ceratonia siliqua* is a concentrate of an infusion of said leaves.

In a preferred embodiment of the first aspect of the invention, the composition is a transdermal patch. Preferably, the transdermal patch comprises an adhesive. Preferably, the transdermal patch comprises a porous membrane. In a preferred embodiment, the porous membrane comprises wood of *Ceratonia siliqua.* In another preferred embodiment, the patch of the present invention comprises wood and bark of *Ceratonia siliqua.* The authors of the present invention have observed that a disc with a diameter of 7 mm and a thickness of 2 mm made from wood of *Ceratonia siliqua* can eliminate the symptoms of haemorrhoids completely when placed in contact with the skin of an affected individual.

In addition, the inventors have found that the disc is equally effective when it comprises bark from *Ceratonia siliqua* around the disc of central wood resulting from a transverse cut of a branch in sections with a thickness of 2 mm.

In a preferred embodiment of the first aspect of the invention, the transdermal patch comprises at least one outer barrier layer and one inner layer comprising the extract. Preferably, the composition comprises water, ethanol, acetone, methanol, or combinations thereof. In a preferred embodiment, the composition comprises acetone. In a preferred embodiment, the composition comprises methanol. In a preferred embodiment, the composition comprises ethanol.

In a preferred embodiment of the first aspect of the invention, the composition also comprises at least one plasticiser. Preferably, the plasticiser is selected from among dibutylphthalate, triethylcitrate, polyethylene glycol, polypropylene glycol, or mixtures thereof.

In a preferred embodiment of the first aspect of the invention, the composition also comprises at least one permeability enhancer.

A second aspect of the present invention refers to a method for the preparation of a composition of the first aspect comprising the following stages:
a. Obtaining an extract from wood, leaves, and/or root of *Ceratonia siliqua;*
b. Diluting the extract obtained in stage (a) in at least one acceptable solvent; and;
c. Loading the diluted extract of stage (b) in a substrate.

In a third aspect the present invention relates to the use of a composition comprising an extract from the wood and/or leaves of *Ceratonia siliqua* for the prevention and/or treatment of haemorrhoids. Preferably, the composition is a transdermal patch.

This invention provides a method for supplying the extract from wood and/or leaves of *Ceratonia siliqua* through the skin of a subject that comprises the administration of a transdermal patch to the skin of said subject.

This invention provides a method for the treatment of a human subject with haemorrhoids comprising the periodic administration of a transdermal patch to said human subject.

This invention provides a transdermal patch as described herein for use in the prevention and/or treatment of a human subject with haemorrhoids.

In a preferred embodiment the transdermal patch also comprises a highly porous membrane. In another preferred embodiment the pharmaceutical composition also comprises an adhesive. In another preferred embodiment, the pharmaceutical composition comprises at least one permeability enhancer. In one embodiment, the transdermal patch is a matrix patch. In another embodiment, the matrix patch comprises an adhesive. In one embodiment, the transdermal patch is a reservoir patch. In another embodiment, the reservoir patch also comprises a highly porous membrane. In another embodiment, the reservoir patch comprises at least one permeability enhancer. In another embodiment, the permeability enhancer is selected from among a group consisting of fatty acid, an alcohol, diethyleneglycol, monoethyl, alpha-tocoferol, a sulphoxide, an azone, a pyrrolidone or a derivative thereof, a terpene, a terpenoid, methyl acetate, butyl acetate, and a cyclodextrin. In another embodiment, the permeability enhancer is oleic acid. In another embodiment, the permeability enhancer is isopropyl miristate. In yet another embodiment, the permeability enhancer is an azone.

In one embodiment, the transdermal patch has a total area of approximately 5-50 cm². In another embodiment, the transdermal patch has a total area of approximately 5- 30 cm². In another embodiment, the transdermal patch has a total area of approximately 5-20 cm². In another embodiment, the transdermal patch has a total area of approximately 5-10 cm². In another embodiment, the transdermal patch has a total area of 5 cm². In another embodiment, the transdermal patch has a total area of 10 cm². In another embodiment, the transdermal patch has a total area of 20 cm².

In one embodiment the coating is a coating of polyethylene terephthalate (PET). In another embodiment, the PET coating is siliconated or has a fluoropolymeric layer. In yet another embodiment, a backup layer comprises a polymer selected from among a group consisting of PET, polypropylene and polyurethane.

This invention also provides a method for supplying an extract from wood and/or leaves of *Ceratonia siliqua* through the skin of a subject that comprises the administration of a transdermal patch to the skin of said subject as described in the present description.

A "transdermal patch" can include, for example, matrix patches and reservoir patches. The matrix patches contain the extract that will be supplied in a semisolid matrix comprising an extract and an adhesive. The reservoir patch comprises a layer, separate from the adhesive, that contains the extract to be supplied.

A "needle patch" is a transdermal patch with small needles that micro-perforate the skin in order to increase permeation or permeability of the extract administered through the barrier. In one embodiment, the transdermal patch described in the present invention is a needle patch.

A "highly-porous membrane" is a membrane with a high permeability to gas, air, and liquid. The membrane parameters that affect its permeability can be, for example, total weight per surface area, thickness, porosity, size of pore for mean flow, and the permeability to air Gurley number (which describes the number of seconds for 100 cubic centimetres to pass through a given material of 6.45 cm² (1.0 square inches) at a differential pressure of 0.013 kg/cm² (4.88 inches of water (0.188 psi) (ISO 5636-5:2003)).

The flow is lower with a higher Gurley number and greater membrane thickness. Other factors affecting flow include the size of the membrane pore and the weight per surface area. In addition, lower Gurley numbers are associated with a greater risk of leaks from the reservoir. Suitable membrane parameters must be selected such that: 1) flow through the membrane is not affected; and 2) an effective barrier is provided to keep the liquid from leading from the reservoir.

In one embodiment the highly porous membrane has the following parameters: 1-20 g/m² total weight per surface area, 8-120 microns thickness, 40-99% pore volume, optionally 75-90% pore volume, 1-200 s/50 ml Gurley number, and up to 1.1 microns mean flow pore size. In another embodiment the highly porous membrane has the following parameters: 3.0-16 g/m² total weight per surface area, 20-120 microns thickness, 80-90% pore volume, 1-5 s/50 ml Gurley number, and 0.3 - 1.1 microns mean flow pore size.

In another embodiment the highly porous membrane has the following parameters: 40-50% pore volume, 8-35 microns thickness, 4-20 g/m² base weight, and 20-200 s/50 ml Gurley number, and < 0.1 microns pore size. In yet another embodiment the highly porous membrane has the following parameters: 75-90% pore volume, 10-120 microns thickness, 3-20 g/m² base weight, 1-100 s/50 ml Gurley number, and 0.05-1.0 microns pore size.

The term "composition" is understood as in a pharmaceutical, nutraceutical or healthcare product composition, and is meant to cover a product comprising an extract from wood and/or leaves of *Ceratonia siliqua* and an inert ingredient or carrier.

The "permeation or permeability enhancers" are agents that increase the bioavailability of the extract. The permeation enhancers include, but are not limited to, fatty acids including oleic acid, propylene glycol, aloe vera oil, isopropyl miristate, n-dodecyl nitrogen heterocyclic heptane-2-ketone, soya oil, diethyilene glycol monoethyl, alpha-tocopherol, alcohol (for example, ethanol or isopropanol), sulphoxide (for example, dimethyl sulphoxide), azones (for example, lauryl caprolactone), pyrrolidone (and derivatives thereof), terpenes, terpenoids, ethyl acetate, methyl acetate, butyl acetate, and cyclodextrins.

In another embodiment, the permeation enhancer is an oleic acid. In another embodiment, the permeation enhancer is an isopropyl miristate. In yet another embodiment, the permeation enhancer is an azone. In one embodiment the permeation enhancers can be up to 15% by weight of the composition.

A "backup layer" is an impermeable flexible cover layer that protects the patch from the external surroundings. A backup layer can be composed of a material such as a polymer that includes but is not limited to PET, polypropylene, and polyurethane.

"Administering to the subject" or "administering to the (human) patient" means delivering, dispensing, or applying the composition of the present invention.

"Treatment" is understood as, for example, inducing the inhibition, regression, or stasis of an ailment or disease, inhibiting, reducing or decreasing the seriousness or substantially eliminating, or improving a symptom of the disease or disorder. In this case the composition of the invention has been shown to completely eliminate the symptoms of persons suffering from haemorrhoids treated with said composition.

A transdermal patch normally consists of a small adhesive bandage containing the drug to be administered. These bandages can come in various forms. The simplest type is a single-part adhesive comprising a reservoir with a drug placed on a support. The reservoir typically consists in a pharmaceutically acceptable pressure-sensitive adhesive, but in some cases it can consist in a non-adhesive material whose area of contact with the skin is provided with a thin layer of a suitable adhesive. Since transdermal patches release the drug by diffusion through the skin, the rate of release of the drug from the patch is governed by Fick's Law and is proportional to the saturation concentration of the drug in the reservoir.

Therefore, the present invention centres on a transdermal device for releasing compounds with antioxidant activity based on particles of *Ceratonia siliqua* for the treatment of *Haimorrhoideis phlebes,* which hitherto has not been recognised as a treatment method nor as a cure for said disease in the scientific literature.

The technical problem solved by this invention is the treatment of the inflammation, pain and itch caused by *Haimorrhoideis Phlebes* with a treatment of *Ceratonia siliqua,* which is non-invasive as the product acts only in contact with the skin and can be placed far from the affected area.

The main advantage provided by this product is that often times conventional treatments for *Haimorrhoideis phlebes* do not work or feel very uncomfortable when used, as well as being made from chemical compounds which can cause serious side effects on prolonged exposure. The product is made from natural extracts and produces immediate relief simply by being in contact with the skin, without requiring the application of a lotion or the ingestion of pills, nor any treatment in the affected area.

An example of a transdermal patch object of the present invention consists in a disc with about 7 mm diameter and 2 mm thickness made from wood of *Ceratonia siliqua* that has been impregnated in an extract from the wood itself to improve its properties. Wood of *Ceratonia siliqua* has been shown to have antihaemorrhoid properties in itself, and discs thereof can be used in contact with the skin to obtain the desired effect. Another example of a transdermal patch of the present invention consists in a porous membrane to which an extract from root, wood, and/or leaves of *Ceratonia siliqua* is applied.

The inclusion of the wood in a patch and the increased concentration of active compounds resulting from impregnation with an extract from the same plant represents an additional advantage, as it allows reducing the size of the device without losing its beneficial effect.

### DESCRIPTION OF THE FIGURES

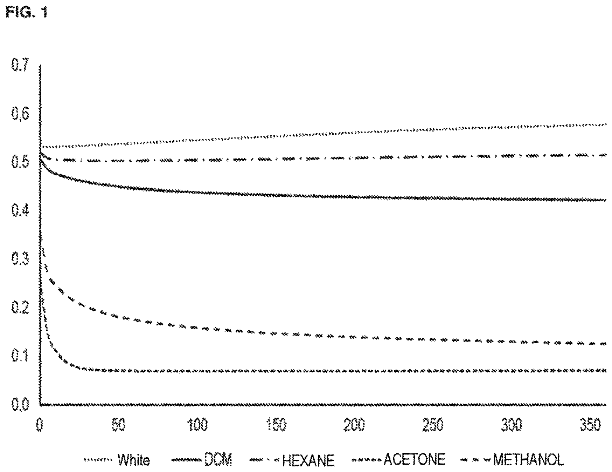

Figure 1. Antioxidant power of *Ceratonia Siliqua* extract measured in times (minutes) on the abscissa and absorbance at 515 nm in the ordinate axis (white, dichloromethane (DCM), hexane, acetone, methanol).

### EXAMPLES

This invention will be better understood with reference to the examples shown below, but it will be obvious to persons skilled in the art that the specific examples indicated are merely illustrative of the invention.

### Example 1: obtaining the extract from wood of Ceratonia siliqua.

The wood of *Ceratonia siliqua* was pulverised to a particle size under 1 mm and the active components were extracted with a 1:1 ethanol/water mixture at room temperature for 24 hours in the dark, with a solid/liquid ratio of 1/10. After this time the extract was filtered with Whatman paper no. 1 and taken to dryness at reduced pressure to a constant weight.

### Example 2: measurement of the antioxidant activity of the extract

The antioxidant activity of the extract obtained in example 1 was analysed with the method described in Brand-Williams, W. et al. (1995) LWT - Food Science and Technology, 28(1), 25-30, adapted as described in Carmona-Jiménez, Y et al. (2014) Food Chemistry 165, 198-204. This activity was tested in dilutions of the extract in various solvents: dichloromethane (DCM), hexane, acetone, and methanol. The results are shown in figure 1. It was observed that the extract diluted in acetone or methanol presented a high antioxidant activity, better than when the other solvents were used. Moreover, these solvents are more environmentally friendly.

### Example 3: preparation of a transdermal patch comprising the extract.

The extract obtained as explained in example 1 was redissolved in ethanol to form a solution of 500 mg of extract/ml and slices of *Ceratonia siliqua* wood 2 mm thick were submerged in it to enrich them in the active components.

The wood was left in contact with the extract for 48 h at room temperature and in the dark. Once impregnated the wood slices were allowed to dry and cut to the desired shape. The impregnated wood discs obtained in this way were placed in direct contact with the skin.

Transdermal patches were prepared with a support layer containing an adhesive element on which was placed a disc with 7 mm diameter and 2 mm thickness of *Ceratonia siliqua* wood impregnated in the aforementioned extract. With the adhesive support the transdermal patch was able to attach to the skin comfortably, so that the impregnated disc of *Ceratonia siliqua* wood was in contact with the skin, allowing the absorption of active compounds of the extract by the organism.

### Example 4: effectiveness of the transdermal patch

The composition of the present invention was tested on 40 adults aged 25 to 75. Of these, 4 presented a more serious case of haemorrhoids and were awaiting treatment by surgery. In 100 % of cases the patients referred a partial improvement 30 minutes after application of the patch and full improvement after one hour. Interestingly, 70% of the subjects on which the patch was tested did not believe in natural medicine, while 100% obtained positive results. The 4 adults with scheduled surgical interventions cancelled them in view of the improvements obtained with the patch of the present invention.

### Example 5: analysis of the composition of the extract of example 1.

The chemical composition of the extract of example 1 was analysed by HPLC as explained in Torres, J. L. et al. (2002) Journal of Agricultural and Food Chemistry, 50(26), 7548-7555; Villaño, D. et al. (2005) Analytica Chimica Acta, 538(1-2), 391-398; Hanan A. Jambi (2015) Life Science Journal 12(12), 1-5.

Table 1 shows the concentration in mg/kg of extract and the relative abundance of a series of compounds in the extract of example 1. Table 2 shows the concentration and relative abundance of flavonoids in the extract of example 1. Table 3 shows the concentration and relative abundance of volatile compounds in the extract of example 1. Finally, table 4 shows the concentrations and relative abundances of some compounds in the patch obtained according to example 3, and how the concentration of these compounds in example 3 has increased with respect to the extract.

**Table 1. Results of polyphenols analysis in the extract from Ceratonia siliqua wood prepared according to example 1.**

| **Compound** | **Concentration (mg/ kg extract)** | **Relative abundance (%)** |
|---|---|---|
| Gallic acid | 328 | 15.33 |
| Pyrogallol | 600 | 28.05 |
| 3-OH-tyrosol | 180 | 8.42 |
| 4-Amino benzoic | 30 | 1.40 |
| Protocatchnic | 75 | 3.51 |
| chlorogenic | 160 | 7.48 |
| catechol | 10 | 0.47 |
| catechin | 85 | 3.97 |
| caffeine | 14 | 0.65 |
| P-OH-benzoic | 40 | 1.87 |
| caffeic | 10 | 0.47 |
| vainillinic | 5 | 0.23 |
| ferrulic | 20 | 0.94 |
| *iso*-ferrulic | 22 | 1.03 |
| *e*-vannilic | 306 | 14.31 |
| resveratrol | 27 | 1.26 |
| *alpha*-cu m aric | 12 | 0.56 |
| benzoic | 150 | 7.01 |
| 3,4,5-methoxycinnamic | 12 | 0.56 |
| sallicilic | 35 | 1.64 |
| cumarin | 16 | 0.75 |
| *p*-cumaric | 2 | 0.09 |

**Table 2. Results of flavonoids analysis in the extract from Ceratonia siliqua wood prepared according to example 1.**

| **Compound** | **Concentration (mg/kg extract)** | **Relative abundance (%)** |
|---|---|---|
| naringin | 14 | 7.78 |
| rutin | 32 | 17.78 |
| hisperidin | 25 | 13.89 |
| rosmarinic acid | 7 | 3.89 |
| quercetrin | 54 | 30.00 |
| quercotin | 4 | 2.22 |
| hispertin | 16 | 8.89 |
| kampferol | 8 | 4.44 |
| apegenin | 19 | 10.56 |
| 7-OH-flavone | 1 | 0.56 |

**Table 3. Results of volatile compounds analysis in the extract from Ceratonia siliqua wood prepared according to example 1.**

| **Compound** | **Concentration (mg/kg extract)** | **Relative abundance (%)** |
|---|---|---|
| ***Hydrocarbons*** | | |
| naphthalene | 0.4 | 0.42 |
| β-cedrene | 7.3 | 7.70 |
| heptadecane | 2.5 | 2.64 |
| eicosene | 20.6 | 21.73 |
| nonadecane | 1.3 | 1.37 |
| heneicosane | 10.4 | 10.97 |

| ***Terpenes*** | | |
|---|---|---|
| limonene | 1.2 | 1.27 |
| camphor | 1.3 | 1.37 |
| nerolidol | 0.5 | 0.53 |
| β-patchoulene | 1.6 | 1.69 |
| farnesol | 15.3 | 16.14 |

| ***Esters*** | | |
|---|---|---|
| tiglate | 0.6 | 0.63 |
| Methyl hexanoate | 0.5 | 0.53 |
| Isoamyl butyrate | 0.7 | 0.74 |
| Geranyl isobutyrate | 3.1 | 3.27 |
| phenylethyl tiglate | 9.4 | 9.92 |

| ***Aldehydes and ketones*** | | |
|---|---|---|
| benzaldehyde | 0.6 | 0.63 |
| 2-hexanone | 1.2 | 1.27 |

| ***Alcohols*** | | |
|---|---|---|
| 3-Hexanol | 0.8 | 0.84 |

| ***acids*** | | |
|---|---|---|
| phthalic | 3.8 | 4.01 |
| hexadecanoic | 6.5 | 6.86 |
| octadecanoic | 5.2 | 5.49 |

**Table 4. Concentration of some compounds in the patch described in example 3.**

| **Compound** | **Concentration (mg/kg extract)** | **Relative abundance (%)** |
|---|---|---|
| Gallic acid | 428 | 16.75 |
| pyrogallol | 650 | 25.44 |
| 3-OH-tyrosol | 190 | 7.44 |
| 4-amino benzoic | 32 | 1.25 |
| Protocatchnic | 78 | 3.05 |
| chlorogenic | 167 | 6.54 |
| catechol | 11 | 0.43 |
| catechin | 87 | 3.41 |
| caffeine | 16 | 0.63 |
| P-OH-benzoic | 43 | 1.68 |
| caffeic | 10 | 0.39 |
| vainillinic | 5 | 0.20 |
| ferrulic | 22 | 0.86 |
| *iso*-ferrulic | 25 | 0.98 |
| *e*-vannilic | 315 | 12.33 |
| resveratrol | 30 | 1.17 |
| *alpha*-cumaric | 15 | 0.59 |
| benzoic | 159 | 6.22 |
| 3,4,5-methoxycinnamic | 12 | 0.47 |
| sallicilic | 40 | 1.57 |
| cumarin | 19 | 0.74 |
| *p*-cumaric | 2 | 0.08 |
| naringin | 15 | 0.59 |
| rutin | 35 | 1.37 |
| hisperidin | 27 | 1.06 |
| rosmarinic acid | 7 | 0.27 |
| quercetrin | 58 | 2.27 |
| quercetin | 4 | 0.16 |
| hispertin | 20 | 0.78 |
| kaempferol | 10 | 0.39 |
| apigenin | 22 | 0.86 |
| 7-OH-flavone | 1 | 0.04 |

## Claims

1. A composition comprising an extract from the wood, leaves, and/or roots of *Ceratonia siliqua* for the prevention and/or treatment of haemorrhoids.

2. The composition according to claim 1, where the extract is of *Ceratonia siliqua* wood.

3. The composition according to claim 1, where the extract is of *Ceratonia siliqua* leaves.

4. The composition according to any of claims 1 to 4, where said composition is a transdermal patch.

5. The composition according to claim 4, where the transdermal patch comprises an adhesive.

6. The composition according to any of claims 4 or 5, where the transdermal patch comprises a porous membrane.

7. The composition according to claim 6, where the porous membrane comprises *Ceratonia siliqua* wood.

8. The composition according to any of claims 4 to 7, where the transdermal patch comprises at least one outer barrier layer and one inner layer comprising the extract.

9. The composition according to any of claims 1 to 8, where said composition comprises water, ethanol, acetone, methanol or combinations thereof.

10. The composition according to claim 9, **characterised in that** it comprises acetone.

11. The composition according to claim 9, **characterised in that** it comprises methanol.

12. The composition according to claim 9, **characterised in that** it comprises ethanol.

13. The composition according to any of claims 1 to 12, which also comprises at least a plasticiser.

14. The composition according to claim 13, where the plasticiser is selected from among dibutylphthalate, triethylcitrate, polyethylene glycol, polypropylene glycol, or mixtures thereof.

15. The composition according to any of claims 1 to 14, which also comprises at least a permeability enhancer.

16. A method for preparing the composition according to any of claims 1 to 15, which comprises the following stages:
a. Obtaining an extract from wood, leaves, and/or root of *Ceratonia siliqua;*
b. Diluting the extract obtained in stage (a) in at least one acceptable solvent; and;
c. Loading the diluted extract of stage (b) in a substrate.
